# EUROPÄISCHE PATENTSCHRIFT

(11) **EP 3 466 253 B1**
(45) Veröffentlichungstag und Bekanntmachung des Hinweises auf die Patenterteilung: **20.07.2022**
(21) Anmeldenummer: 18199226.4
(22) Anmeldetag: 09.10.2018
(51) Int. Cl.: A01K 45/00, A01K 29/00, G01V 8/20

(54) **VORRICHTUNG FÜR DIE AUFFINDUNG VON OBJEKTEN IN EINEM TIERSTALL**
DEVICE FOR THE DETECTION OF OBJECTS IN AN ANIMAL STABLE
DISPOSITIF DE LOCALISATION DES OBJETS DANS UNE ÉTABLE D'ANIMAL

(30) Priorität: 09.10.2017 DE 202017106101 U
(43) Veröffentlichungstag der Anmeldung: 10.04.2019
(73) Patentinhaber: Big Dutchman International GmbH, 49377 Vechta (DE)
(72) Erfinder: KÜKING, Jörg, 49377 Vechta (DE)
(74) Vertreter: Eisenführ Speiser

(56) Entgegenhaltungen:
- EP-A1- 2 698 763
- EP-A2- 1 736 801
- WO-A1-99/09910
- WO-A1-2015/121431
- DE-A1- 2 810 891
- DE-A1-102008 035 888
- US-A1- 2016 363 692

## Beschreibung

Die Erfindung betrifft eine Vorrichtung zur Erfassung kranker oder toter Tiere in einem Geflügelstall, umfassend: eine berührungslos arbeitende Abtastvorrichtung, die ausgebildet ist, um eine elektromagnetische Strahlung in einer Abtastrichtung auszusenden und eine Reflexion der elektromagnetischen Strahlung zu empfangen, eine mit der Abtastvorrichtung signaltechnisch verbundene elektronische Auswertungseinheit, die ausgebildet ist, um die von der Abtastvorrichtung empfangenen Signale auszuwerten.

In der heutigen Nutztierhaltung werden typischerweise mehrere Tiere gemeinsam in einer Tierhaltungseinheit gehalten, beispielsweise bei der Haltung von Legehennen, Broilern oder allgemein Geflügelnutztieren in Käfighaltung, Volierenhaltung oder in mehretagigen Haltungssystemen. Viele Vorgänge dieser Nutztierhaltung sind automatisiert, beispielsweise die Zufuhr von Futtermittel und Flüssigkeit, gegebenenfalls auch Maßnahmen, die der Hygiene dienen, wie Kotentfernung und weitere Maßnahmen, wie beispielsweise die Einsammlung von Eiern.

Aus hygienischen Gründen ist es wünschenswert, dass Gegenstände, die diese automatisierten Abläufe stören, entfernt werden, bevor hierdurch hygienische Probleme auftreten. So kommt es beispielsweise vor, dass auf einer Oberfläche im Bereich eines Haltungssystems, die dem Abrollen von Eiern zur Eiersammlung dient, ein dort nicht vorgesehener Gegenstand liegt, an dem die Eier sich sammeln oder der eine Beschädigung der Eier beim Abrollen verursacht. Dies kann die Produktqualität und die Produktfrische beeinträchtigen.

Aus EP 1 212 939 A1 ist ein System zur Überwachung einer Farm mithilfe von Kameras vorbekannt. Das System arbeitet mit mehreren Videokameras, deren Bildsignale mittels einer computergesteuerten Auswertung analysiert werden, um hieraus den Aufenthaltsort der Tiere abzuleiten.

Aus EP 2260699 B1 und EP 2786655 B1 sind hierzu fortschrittliche Systeme zur Überwachung eines Nutztierbestandes vorbekannt, die ebenfalls mittels einer Videokamera-überwachung den Aufenthaltsort und darüber hinaus Verhaltensweisen der Tiere in einer sicheren und automatisierten Weise ermöglichen.

Diese vorbekannten, auf Grundlage einer laufenden Video-Überwachung basierenden Systeme eignen sich zwar auch im Allgemeinen dazu, Tiere im Tieraufenthaltsbereich zu erkennen, sind jedoch für die wirtschaftliche Überwachung von Nutztierbeständen auf unvorhergesehene Gegenstände in vielen Haltungsformen nicht geeignet. Diese fehlende Eignung liegt zum einen darin, dass die Systeme auf eine Übersicht-Überwachung aufbauen, die in vielen Haltungsformen aufgrund räumlicher Bedingungen nicht gewährleistet werden kann, zum anderen sind die notwendigen Komponenten, die zur Durchführung der vorbekannten Überwachungsverfahren erforderlich sind, sowohl hinsichtlich der Investitionskosten als auch der Wartung zu aufwendig, um eine wirtschaftliche und zuverlässige Überwachung von Nutztierbeständen im Hinblick auf Gegenstände, die nicht im Tierhaltungsbereich vorgesehen sind, zu ermöglichen.

Aus WO 2015/121431 A1 ist eine Vorrichtung und ein Verfahren vorbekannt, die zur Erfassung toter Tiere in einem Stall dient. Die Vorrichtung verwendet einen zweidimensionalen Laserscanner, der mittels eines verschwenkbaren Lasers einen Winkelbereich erfasst und bei Reflexion durch einen Körper den Winkel und den Abstand auf Grundlage der Laufzeit nach dem Prinzip des Sonars bestimmt. Der Laserscanner wird dabei gemäß dieses Standes der Technik so eingesetzt, dass ein Mapping von zwei oder mehr unterschiedlichen Punkten ausgeführt wird und hierbei Objekte anhand der Reflexion der Laserstrahlung ermittelt werden. Die Mappings werden mit zweidimensionalen Scanmitteln ausgeführt, die auf der Höhe der Beine der Nutztiere angeordnet sind. Wenn ein Nutztier in zwei im zeitlichen Abstand aufeinanderfolgenden Mappings in der gleichen Position identifiziert wird, wird hieraus ein Signal erzeugt, welches das Vorhandensein eines bewegungslosen Tieres anzeigt, woraus auf ein totes Tier geschlossen wird.

Grundsätzlich ist es mit einem solchen zweidimensionalen Laser-Scanning-System in technisch anderer Weise möglich, tote Tiere in einem Stall zu ermitteln als mit videobasierten Systemen. Allerdings ist es für eine einerseits schnelle und andererseits zuverlässige Ermittlung der toten Tiere notwendig, dass System zu verbessern, um die Tierüberwachung effizient und ohne zusätzliche Belastung der Tiere durchzuführen. Unter Berücksichtigung der Tierbelastung kann mit einem solchen System eine Erkennung toter Tiere nur nach ca. 24 Stunden gewährleistet werden.

Als ein weiteres Problem des Standes der Technik ergibt sich, dass gemäß WO 2015/121431 A1 der Laserscanner an einem Futterwagen montiert ist und mit diesem Futterwagen horizontal entlang einer mehrstöckigen Käfigreihe verfahren wird. Dies macht es erforderlich, dass zur Erfassung der mehreren Stockwerke der Käfigreihen eine zusätzliche vertikale Verschiebung des Laserscanners durchgeführt wird. Durch diese sowohl horizontale als auch vertikale Verschiebung werden die Nutztiere in den Käfigen einem zusätzlichen Stress ausgesetzt, der durch die mehrmalige Vorbeifahrt des Futterwagens, die damit geweckte Futtererwartungshaltung und deren Enttäuschung ausgelöst wird. Eine Anordnung, bei der auf jeder Etage des Stalls ein Laserscanner vorgesehen ist, kann zwar die Anzahl der notwendigen Verfahrbewegungen reduzieren, löst aber als damit verbundenen Nachteil erheblich zusätzliche Kosten aufgrund der notwendigen Laserscanner und entsprechender Auswertungseinheiten aus.

Eine weitere Problematik liegt in der praktischen Ortsbestimmung eines aufgefundenen toten Tieres. Zwar wird in WO 2015/121431 A1 der Vorteil einer Ortsbestimmung eines aufgefundenen toten Tieres grundsätzlich beschrieben, jedoch stellt sich diese Ortsbestimmung in der tatsächlichen Verwirklichung als problembehaftet dar. Dies liegt zum einen grundsätzlich daran, dass Haltungssysteme für Nutztiere in der Regel einer individuellen Planung und Dimensionierung unterliegen und daher jegliche Ortsangaben für ihre Verarbeitung und Ableitung einer entsprechenden Konfiguration bedürfen, um eine an das individuelle Haltungssystem angepasste und zutreffende Ortsangabe zu ermöglichen. Aufgrund der Umgebungsbedingungen werden zur Ortsermittlung notwendige Messwerte zudem häufig über einen längeren Benutzungszeitraum verschoben oder verfälscht und sind daher keine zuverlässige Angabe bei einem praktischen Einsatz des Systems über einen längeren Zeitraum. Schließlich erfordert die Ermittlung einer Ortsangabe regelmäßig das Abfahren des gesamten Haltungssystems mit dem Laserscanner und die Auswertung der zweidimensionalen Scandaten von zumindest zwei Positionen. Hierdurch wird eine punktuelle Überprüfung und räumliche Zuordnung von Objekten, die einer engermaschigen Kontrolle unterliegen sollen, sowohl hinsichtlich der Durchführung des Scans als auch hinsichtlich der räumlichen Zuordnung aufwendig und für die Tiere belastend.

Das Dokument US 2016/363692 A1 beschreibt im Allgemeinen ein optisches Viehzählsystem und -verfahren und insbesondere eine nicht-invasive Erkennung und Zählung von Vieh, beispielsweise zur Schlachtung.

Das Dokument DE 102008035888 A1 offenbart eine Einrichtung zum Detektieren von Objekten, wie Tieren und Vogelgelegen, im Acker und Pflanzenbau mit mindestens einer zum Boden ausgerichteten Detektionseinrichtung.

Das Dokument EP 1736801 A2 offenbart eine Einrichtung zum Feststellen und Auffinden von sich in Wiesen aufhaltenden Tieren, wie Rehe, Rehkitze, Hasen mittels einer Sensoranordnung die eine Anzahl von außerhalb einer zu bearbeitenden Wiesenfläche ortsfest und in entsprechender Höhe über der Wiesenfläche angeordnete Sensoreinheiten aufweist.

Das Dokument DE 2810891 A1 offenbart eine Anlage zur Koliküberwachung von Großtieren, insbesondere Pferden, die in Boxen gehalten werden, wobei einer im Abstand zum Boden die gesamte Grundrissfläche der Boxe überbrückenden Schar von Lichtschranken ein zeitabhängiges Zählwerk zugeordnet ist, welches bei einer vorgebbaren Anzahl von Unterbrechungen, wenigstens einer der Lichtschranken innerhalb einer gleichfalls vorgebbaren Zeiteinheit einen eine entfernt angeordnete Alarm- oder Anzeigeeinrichtung auslösenden Impuls erzeugt.

Der Erfindung liegt daher die Aufgabe zugrunde, ein System zur Erfassung von Gegenständen in einem Tierhaltungssystem bereitzustellen, welches in praktisch nutzbarer und wirtschaftlicher Weise eine sichere und schnelle Erkennung solcher Gegenstände erzielt.

Diese Aufgabe wird erfindungsgemäß gelöst mit einer Erfassungsvorrichtung nach Anspruch 1.

Erfindungsgemäß ist die Abtasteinheit dabei so ausgebildet, um in einer vorbestimmten Richtung einen elektromagnetischen Abtaststrahl auszusenden, und eine Reflexion dieses Abtaststrahls aus der vorbestimmten Richtung zu empfangen. Die Abtasteinheit umfasst folglich einen Sender und einen Empfänger für die elektromagnetische Strahlung, die so ausgerichtet sind, dass sie eine Messung in der vorbestimmten Richtung durchführen. Entgegen des Standes der Technik wird mit der erfindungsgemäßen Vorrichtung folglich keine Abtastung von einem Messpunkt ausgehend in einem Winkelbereich durchgeführt, also in mehreren Richtungen abgetastet, sondern die Abtastung erfolgt in genau einer vorbestimmten Richtung. Diese Abtastung in einer Richtung wirkt funktionell mit einer Verfahrbewegung der Abtasteinheit zusammen, die entlang des zu untersuchenden Bereichs auf der Abtastebene für den entsprechenden Erfassungszeitraum bewegt werden kann, um während einer konstanten Bewegung die Abtasteinheit mit ihrer fixierten eindimensionalen Abtastvorrichtung zu bewegen. Auf diese Weise kann aus den während der Bewegung über den Zeitraum der Bewegung empfangenen Laufzeitsignalen ein Laufzeitprofil für die Abtasteinheit erstellt werden. Ergebnis der so eingesetzten Erfassungsvorrichtung ist dann ein Laufzeitprofil aus der Abtasteinheit, welches die jeweils erfassten Laufzeiten beispielsweise als Messpunkte oder als Messkurve über die Zeit oder über den Verfahrweg aufträgt.

Anhand eines solchen Laufzeitprofils kann jedes durch Reflexion erkannte Objekt, das von dem verfahrenen elektromagnetischen Strahl erfasst wurde und die Strahlung reflektiert hat, in seiner Entfernung zu der Abtasteinheit abgelesen werden. Anhand eines solchen Laufzeitprofils kann dann ein Gegenstand, der sich im Tieraufenthaltsbereich befindet, identifiziert und dessen Entfernung kurzfristig ermöglicht werden. Neben der grundsätzlich möglichen Absolutbestimmung solcher Gegenstände, kann dies auch in einer Relativbestimmung - also anhand eines Vergleichs - erfolgen. In diesem Fall wird das Laufzeitprofil mit einem Kalibrierungslaufzeitprofil verglichen, das von dem Tieraufenthaltsbereich in einem Zustand ermittelt wurde, in dem keine Gegenstände oder Fremdkörper in dem Tieraufenthaltsbereich sich befanden. Dieses Kalibrierungslaufzeitprofil wird durch einen Abtastvorgang mit der Abtasteinheit bei leerem Tierhaltungssystem gewonnen. Es dient dann als Referenz, um jegliche demgegenüber hinzugekommenen Gegenstände im Vergleichswege zu erkennen.

Die erfindungsgemäße Vorrichtung kann auch zur Erfassung zeitlicher Veränderungen während der Nutzung des Tierhaltungssystems eingesetzt werden. So kann beispielsweise bei einer in zeitlicher Beabstandung durchgeführten zweimaligen Abtastung ein und desselben Bereichs, beispielsweise indem die Abtasteinheit zu einem ersten Zeitpunkt und dann zeitversetzt zu einem zweiten Zeitpunkt entlang einer Messstrecke verfahren wird und hierbei jeweils ein entsprechendes Laufzeitprofil erfasst wird, ermittelt werden, ob sich durch die Abtastung erfasste Objekte zwischen der ersten und zweiten Messung bewegt haben oder nicht und bei Erkennung nicht bewegter Objekte darauf geschlossen werden, dass ein unbewegliches Objekt - also ein nicht vorgesehener Gegenstand - erfasst wurde. Weiterhin vorteilhaft ist, dass durch die eindimensionale Messung entlang der Abtastvorrichtung in Verbindung mit einer Verfahrbewegung eine direkt mit der Verfahrbewegung zusammenhängende Erfassung der geometrischen Abmessung eines Objekts in Verfahrrichtung ermöglicht wird. Mit der erfindungsgemäßen Erfassungsvorrichtung wird es daher möglich, in einerseits zuverlässiger und auch unter schmutzbelasteten Umgebungsbedingungen genauen und sicheren Weise, eine wirtschaftlich effiziente Kontrolle von Tierhaltungssystemen durchzuführen und hierbei unerwünschte Gegenstände, die sich im Käfig befinden und beispielsweise den Eierablauf und das Einsammeln der Eier behindern, zu erkennen und eine rechtzeitige Entfernung zu ermöglichen, bevor durch solche Gegenstände zeitliche Verzögerungen bei der Eiereinsammlung auftreten

Die erfindungsgemäße Vorrichtung eignet sich dabei überraschend auch dazu, ein krankes oder totes Tier im Tieraufenthaltsbereich zu erfassen. Entgegen der Lehre des Standes der Technik ist dazu nach Erkenntnis der Erfinder keine aufwendige zweidimensionale Abtastung erforderlich, um etwa Tierkonturen zu erkennen. Stattdessen kann auch mittels der erfindungsgemäßen Abtastung in einer vorbestimmten Abtastrichtung erkannt werden, ob sich ein krankes oder totes Tier auf dem Boden befindet - beispielsweise, indem dieses Tier anhand eines Vergleichs mit einem Kalibrierungsprofil erkannt wird oder anhand eines Vergleichs von zwei zeitlich beabstandeten Abtastungen erkannt wird.

So können kranke oder tote Tiere, die sich im Tieraufenthaltsbereich befinden, zu einem frühen Zeitpunkt erkannt werden, damit diese aus dem Tieraufenthaltsbereich entfernt werden können, bevor eine etwaige Gefährdung der anderen Nutztiere auftritt.

Dabei ist es besonders bevorzugt, wenn die Abtastvorrichtung mindestens zwei vertikal beabstandete Abtasteinheiten umfasst, von denen jede Abtasteinheit einen Sender zum Aussenden eines elektromagnetischen Abtaststrahls in einer vorbestimmten Richtung und einen Empfänger zum Empfang einer Reflexion des Abtaststrahls aus der vorbestimmten Richtung umfasst, und die Auswertungseinheit ausgebildet ist, um aus dem Empfang eines von jeder Abtasteinheit ausgesendetem und reflektierten Signals und dessen Laufzeit ein Laufzeitsignal zu bestimmen und um aus den über einen Zeitraum empfangenen Laufzeitsignalen ein Laufzeitprofil für jede der Abtasteinheiten zu erstellen.

Gemäß dieser Fortbildung werden zumindest zwei Abtasteinheiten bereitgestellt, die Bestandteil der Abtastvorrichtung sind. Die zwei Abtasteinheiten sind vertikal voneinander beabstandet und folglich angeordnet, um, auf unterschiedlichen Höhen in Bezug auf die Schwerkraftrichtung eine Messung durchzuführen. Mit dieser Anordnung wird erreicht, dass mittels der erfindungsgemäßen Abtastvorrichtung zumindest zwei Etagen, in denen Nutztiere gehalten werden, mittels der Abtastvorrichtung in einem Erfassungsvorgang abgetastet und damit auf kranke oder tote Tiere kontrolliert werden können. Das ansonsten notwendige mehrmalige Abfahren unter gleichzeitiger vertikaler Verfahrung einer Abtasteinheit nach einem ersten Durchgang zur Durchführung eines zweiten Abtastvorgangs in einem zweiten Durchgang kann hierdurch entfallen.

Die vorstehend erläuterte Abtastung in genau einer Richtung kann funktionell mit den zwei oder mehreren bereitgestellten Abtasteinheiten zusammenwirken, die in einer gemeinsamen Verfahrbewegung entlang des zu untersuchenden Bereichs auf den zwei Ebenen für den entsprechenden Erfassungszeitraum bewegt werden können, um während einer konstanten Bewegung die Abtasteinheiten mit ihrer fixierten eindimensionalen Abtastvorrichtung zu bewegen. Auf diese Weise kann aus den während der Bewegung über den Zeitraum der Bewegung empfangenen Laufzeitsignalen ein Laufzeitprofil für die beiden und gegebenenfalls weitere vorgesehene Abtasteinheiten jeweils erstellt werden. Dies ermöglicht eine für die Nutztiere weniger belastende Durchführung der Erfassung. Ergebnis der so eingesetzten Erfassungsvorrichtung ist dann ein erstes Laufzeitprofil aus der einen Abtasteinheit, welches die jeweils erfassten Laufzeiten beispielsweise als Messpunkte oder als Messkurve über die Zeit oder über den Verfahrweg aufträgt und ein entsprechend zweites Laufzeitprofil, welches die jeweils erfassten Laufzeitsignale der zweiten Abtasteinheit über die Zeit beziehungsweise über den Verfahrweg aufträgt. Die Belastung der einzelnen Tiere des Tierbestands in der Tierhaltungsvorrichtung durch den Erfassungsvorgang wird hierbei minimiert und der Zeitraum, der für die sichere Erfassung eines kranken oder toten Tieres erforderlich ist, kann gegenüber dem Stand der Technik ohne zusätzliche Belastung der Tiere verkürzt werden.

Gemäß einer ersten bevorzugten Ausführungsform ist vorgesehen, dass die Auswertungseinheit einen elektronischen Laufzeitprofilspeicher umfasst und weiterhin dazu ausgebildet ist, um
- In dem Laufzeitprofilspeicher mindestens ein vorbestimmtes Laufzeitprofilmuster und einer diesem vorbestimmten Laufzeitprofilmuster zugeordnete Ortsangabe abzuspeichern,
- aus einer Mehrzahl von über einen Abtastzeitraum ermittelten Laufzeitsignalen ein Laufzeitprofil zu erstellen, welches die Laufzeitsignale während des Abtastzeitraums darstellt,
- mindestens einen Ausschnitt des Laufzeitprofils mit dem in dem Laufzeitprofilspeicher gespeicherten vorbestimmten Laufzeitprofilmuster zu vergleichen, und
- bei festgestellter Übereinstimmung des mindestens einen Ausschnitts des Laufzeitprofils mit dem Laufzeitprofilmuster dem Ausschnitt aus dem Laufzeitprofil die im Laufzeitprofilspeicher zu dem vorbestimmten Laufzeitprofilmuster gespeicherte Ortsangabe zuzuordnen.

Hierbei ist zu verstehen, dass diese Fortbildung auch unabhängig von der zuvor erläuterten Erfindung mit der Erfassungsvorrichtung der eingangs beschriebenen Art ausgeführt sein kann. Mit diesem Aspekt der Erfindung wird eine einfache und zugleich besonders zuverlässige Möglichkeit bereitgestellt, eine präzise Ortsangabe für ein bestimmtes, in dem Laufzeitprofil erkanntes Objekt, wie beispielsweise ein erkranktes oder totes Tier, zu bestimmen. Das Prinzip der Erfindung beruht dabei darauf, dass bei der Erfassung von Laufzeitsignalen und der Erstellung eines Laufzeitprofils hieraus in den typischen Anwendungsfällen bei Überwachung eines Stallbereichs nicht nur Tiere oder Körperteile von Tieren abgetastet werden, sondern auch feststehende Einbauten und Rahmenelemente der Stalleinrichtung erfasst werden. Diese feststehenden Elemente ergeben ein typisches, bei jeder Messung wiederkehrendes Muster in dem Laufzeitprofil und werden erfindungsgemäß zur Ermittlung einer Ortsangabe herangezogen. Durch Vergleich der Laufzeitsignale, die von diesen feststehenden Elementen ermittelt werden, kann im Laufzeitprofil ein bestimmtes Laufzeitsignal, das ein Tier wiedergibt, eine präzise Ortszuordnung erfahren. Dies wird erfindungsgemäß in praktischer Hinsicht dadurch erzielt, dass ein Laufzeitprofilmuster zum Vergleich herangezogen wird, welches diese feststehenden Elemente wiedergibt und ein vollständiger oder abschnittsweiser Vergleich des jeweils bei einem Messvorgang ermittelten Laufzeitprofils mit diesem Laufzeitprofilmuster durchgeführt wird. Durch Wiedererkennen und Zuordnen der Laufzeitsignale im Laufzeitprofil, die die feststehenden Elemente, die im Laufzeitprofilmuster wiedergegeben sind, darstellen, kann die Zuordnung einer Ortsangabe in einer zuverlässigen und präzisen Weise erfolgen.

Dieser Aspekt und diese Fortbildung der Erfindung vermeidet die Problematik, die sich aus der Verwendung von Wegsensoren, Abstandsmesseinrichtungen oder aus Motorumdrehungen abgeleiteten Ortsangaben ergibt, wenn diese in schmutzbelasteten Umgebungen eingesetzt werden und dadurch durch Verschmutzung, Durchrutschen, Blockaden oder unregelmäßige Bewegungsweisen auftretende Ungenauigkeiten und Messwertverfälschungen die Ortsangaben unpräzise und unzuverlässig machen. Anstelle solcher kostenverursachender zusätzlicher Komponenten und Verfahrensschritte zur Ortsangabenermittlung wird gemäß diesem Aspekt und dieser Fortbildung der Erfindung auf die eigentlichen Messdaten zurückgegriffen und diese anhand eines Vergleichs mit Referenzdaten, die stationäre Elemente in der Stalleinrichtung wiedergeben, die Ortsangabe zuverlässig ermittelt. Die Fortbildung und Ausgestaltung dieses Aspekts der Erfindung eignet sich uneingeschränkt dafür, eine Gesamterfassung mit klarer Ortszuordnung durchzuführen, kann aber ebenso genau und in der Auswertung schnell und einfach dazu verwendet werden, nur einen Ausschnitt für eine abschnittsweise Überprüfung zu untersuchen und hinsichtlich der Ortsangabe zuzuordnen, oder innerhalb eines solchen Abschnitts eine bestimmte Struktur einem Ort mit einer entsprechenden Ortsangabe zuzuordnen.

Dabei ist bei dieser Fortbildung und diesem Aspekt der Erfindung unter einem Laufzeitprofilmuster beispielsweise ein Diagramm nach Art einer Messpunkt- oder Messkurvendarstellung zu verstehen, in dem die Laufzeitsignale über die Zeit oder den Weg aufgetragen sind, ebenso wie in einem Laufzeitprofil. Laufzeitprofilmuster und Laufzeitprofil sind dadurch unmittelbar miteinander vergleichbar und vorzugsweise hinsichtlich Einheit und Skalierung der Achsen übereinstimmend. Hierdurch kann, für die Laufzeitsignale, die auf der Y-Achse aufgetragen werden, ein direkter Vergleich erfolgen und feststehende Komponenten der Stalleinrichtung unmittelbar im Laufzeitprofil identifiziert werden und als Referenzpunkt für eine Ortsangabe herangezogen werden.

Unter einem Vergleich von mindestens einem Ausschnitt des Laufzeitprofils mit dem in dem Laufzeitprofilspeicher gespeicherten vorbestimmten Laufzeitprofilmuster, ist hierbei zu verstehen, dass ein, zwei, oder mehr Ausschnitte oder das gesamte Laufzeitprofil mit entsprechend einem, zwei, oder mehr Ausschnitten des Laufzeitprofilmusters oder dem gesamten Laufzeitprofilmuster verglichen werden. Insbesondere kann auch ein Abschnitt oder mehrere Abschnitte des Laufzeitprofils mit dem gesamten Laufzeitprofilmuster verglichen werden, um darin die korrespondierenden Abschnitte, oder den korrespondierenden Abschnitt aufzufinden.

Die vorgenannte Fortbildung, beziehungsweise der vorgenannte Aspekt der Erfindung kann weiter fortgebildet werden, indem die Auswertungseinheit ausgebildet ist, um
- bei dem Vergleich des mindestens einen Ausschnitt des Laufzeitprofils mit dem in dem Laufzeitprofilspeicher gespeicherten vorbestimmten Laufzeitprofilmuster einen ersten Laufzeitprofilabschnitt, einen zweiten Laufzeitprofilabschnitt und einen Ortsabstand, der eine räumliche Distanz zwischen dem ersten Laufzeitprofilabschnitt und dem zweiten Laufzeitprofilabschnitt definiert, mit einem ersten Laufzeitprofilmusterabschnitt des Laufzeitprofilmusters, einem zweiten Laufzeitprofilmusterabschnitt des Laufzeitprofilmusters und einem vorbestimmten Ortsabstand, der eine Distanz zwischen dem ersten Laufzeitprofilmusterabschnitt und dem zweiten Laufzeitprofilmusterabschnitt definiert, zu vergleichen, und
- bei festgestellter Übereinstimmung des ersten Laufzeitprofilabschnitts mit dem ersten Laufzeitprofilmusterabschnitt und festgestellter Übereinstimmung des zweiten Laufzeitprofilabschnitts mit dem zweiten Laufzeitprofilmusterabschnitt und festgestellter Übereinstimmung des Ortsabstands mit dem vorbestimmten Ortsabstand dem Laufzeitprofil die in dem Laufzeitprofilspeicher zu dem vorbestimmten Laufzeitprofilmuster gespeicherte Ortsangabe zuzuordnen.

Bei dieser Fortbildung ist die Auswertungseinheit solcherart ausgebildet, dass sie anhand von zwei abgespeicherten Laufzeitprofilabschnitten und einem Abstand, der zwischen diesen Laufzeitprofilabschnitten vorliegt und ebenfalls abgespeichert ist, den Vergleich durchführt. Diese Vergleichsmöglichkeit bietet eine besonders zuverlässige und genaue Bestimmung der Lokalisation eines Laufzeitprofils beziehungsweise eines Punktes in einem Laufzeitprofil. Werden in dem Laufzeitprofil zwei Laufzeitprofilabschnitte aufgefunden, die den entsprechenden Laufzeitprofilmusterabschnitten entsprechen und in dem gleichen Abstand voneinander angeordnet sind wie in dem Laufzeitprofilmusterabschnitt, so kann eine entsprechende Ortsangabe eindeutig zugeordnet werden und ausgehend von dieser Ortsangabe mit entsprechenden Abstandsberechnungen jeder Ort, bzw. Messpunkt in dem Laufzeitprofil einem entsprechend bestimmen Ort in der überwachten Tierhaltungseinrichtung zugordnet werden.

Erfindungsgemäß ist vorgesehen, dass die Auswertungseinheit ausgebildet ist, um
- In einem Kalibrierungsmodus ein erstes Laufzeitprofil aufzunehmen und das erste Laufzeitprofil als Laufzeitprofilmuster in dem Laufzeitprofilspeicher abzuspeichern, und
- In einem Überwachungsmodus ein zweites Laufzeitprofil und nachfolgend gegebenenfalls weitere Laufzeitprofile aufzunehmen und dieses zweite bzw. jedes weitere Laufzeitprofil mit dem Laufzeitprofilmuster zu vergleichen.

Demnach wird zunächst in einem Kalibrierungsmodus eine Messung durchgeführt, indem der zu überwachende Abschnitt der Tierhaltungsvorrichtung mit der Abtasteinrichtung abgefahren wird und ein Laufzeitprofil ermittelt wird. Dieses Laufzeitprofil kann die gesamte Messstrecke umfassen oder kann einzelne Abschnitte oder einen einzigen Abschnitt dieser Messstrecke umfassen. Das Laufzeitprofil wird dann als Laufzeitprofilmuster abgespeichert und dient nachfolgend als Referenz für den Vergleich weiterer aufgenommener Laufzeitprofile. Insbesondere kann im Kalibrierungsmodus das Laufzeitprofil aufgenommen werden, wenn die Tierhaltungseinrichtung nicht mit Tieren belegt ist, sodass ausschließlich die feststehenden Einrichtungen der Stalleinrichtung bei dem Laufzeitprofil erfasst werden.

Noch weiter ist es bevorzugt, wenn die erfindungsgemäße Vorrichtung durch eine Verfahreinrichtung zum Verfahren der Abtastvorrichtung entlang einer Tierhaltungseinrichtung in einer Verfahrrichtung, die quer, insbesondere senkrecht zur Abtastvorrichtung ausgerichtet ist, fortgebildet ist. Eine solche Verfahreinrichtung ermöglicht es, die Abtasteinrichtung entlang einer Kurve oder einer Geraden oder einer sonstigen Bahn an den zu überwachenden Tieren vorbeizuverfahren. Die Verfahrrichtung ist dabei vorzugsweise quer, insbesondere senkrecht zur Abtastvorrichtung ausgerichtet, sodass unter der laufenden Verfahrbewegung mit der elektromagnetischen Strahlung stets neue Bereiche abgetastet werden und die für einen räumlichen Scanvorgang notwendige Scanbewegung durch die Verfahrbewegung bereitgestellt wird. Bei einer typischen Bewegung der Abtasteinrichtung entlang einer geradlinigen Verfahrkurve ergibt sich folglich eine Vielzahl von parallel zueinander liegenden Abtastvorrichtungen, aus denen ein Laufzeitprofil erstellt wird. Das Laufzeitprofil kann dabei typischerweise auf der X-Achse die Verfahrbewegung und auf der Y-Achse das jeweilige Laufzeitsignal aufzeigen und hierdurch einen direkten Rückschluss auf die e der Abtastung erfassten Tiere und Gegenstände ermöglichen.

Grundsätzlich ist zu verstehen, dass die Verfahreinrichtung alle notwendigen Bestandteile einer Erfassungsvorrichtung der erfindungsgemäßen Art bewegen kann, insbesondere solcherart, dass die Abtastvorrichtung mit allen Abtasteinheiten und der Auswertungseinheit sowie einer unabhängigen Energieversorgung hierfür verfahren werden und diese folglich eine unabhängige, autarke Erfassungsvorrichtung darstellen. Die Verfahreinrichtung kann insbesondere an einer auch einem anderen Zweck dienenden Stalleinrichtungseinheit angeordnet sein, beispielsweise einer Fütterungsvorrichtung, einer Reinigungsvorrichtung oder dergleichen, sodass eine ohnehin vorhandene Verfahreinrichtung und deren ohnehin notwendiger Betrieb auch für die Überwachung des Tierbestandes mittels der erfindungsgemäßen Erfassungsvorrichtung genutzt wird.

Gemäß einer noch weiteren bevorzugten Ausführungsform ist vorgesehen, dass die Auswertungseinheit ausgebildet ist, um ein Laufzeitprofil während einer kontinuierlichen Bewegung der Abtasteinrichtung entlang der Tierhaltungseinrichtung zu erfassen, wobei die Tierhaltungseinrichtung vorzugsweise eine Mehrzahl von separaten, voneinander abgetrennten Tierhaltungseinheiten aufweist, und die Auswertungseinheit ausgebildet ist, um die Mehrzahl der separaten, voneinander abgetrennten Tierhaltungseinheiten während der Verfahrbewegung in einem einzigen Laufzeitprofil zu erfassen und mit einem für die Mehrzahl der separaten, voneinander abgetrennten Tierhaltungseinheiten abgespeicherten Laufzeitprofilmuster zu vergleichen. Gemäß dieser Ausführungsform erfolgt die Überwachung mittels der Abtasteinrichtung während einer kontinuierlichen Bewegung der Abtasteinrichtung. Dies kann vorzugsweise entlang einer Mehrzahl von separaten Tierhaltungseinheiten, beispielsweise einzelnen Ställen, Volieren oder Stalleinheiten erfolgen. Die dabei ermittelten Laufzeitsignale werden in einem Laufzeitprofil durch die Auswertungseinheit abgebildet und durch die Auswertungseinheit ausgewertet. Die Ausführungsform ermöglicht einerseits eine für die Tiere nicht belastende Überwachung, da unruheerzeugende Stopp- und Anlaufvorgänge durch die kontinuierliche Bewegung vermieden werden. Weiterhin wird eine effiziente Auswertung ermöglicht, indem ein einziges Laufzeitprofil für eine Käfigreihe beziehungsweise von Tierhaltungseinrichtungen erstellt und ausgewertet wird. Dies erleichtert insbesondere eine präzise Bestimmung einer Ortsangabe zur Lokalisation eines aufgefundenen Tieres, beispielsweise anhand eines Laufzeitprofilmusters. Die Fortbildung macht sich hierbei zunutze, dass die Mehrzahl der überwachten und in einem Verfahrvorgang erfassten Tierhaltungseinrichtungen stationär zueinander angeordnet sind und daher eine zuverlässige Ortsangabe bereits anhand eines einzigen übereinstimmenden Abschnitts eines Laufzeitprofils zu einem Laufzeitprofilmuster erfolgen kann.

Noch weiter ist es bevorzugt, wenn in Verfahrrichtung um einen Abstand beabstandet und in Bezug auf die Verfahrrichtung fluchtend zu einer Abtasteinheit eine Nachbarabtasteinheit angeordnet ist, wobei
- Die Auswertungseinheit ausgebildet ist, um aus dem Empfang eines von der Nachbarabtasteinheit ausgesendeten Signals und dessen Laufzeit ein Nachbarlaufzeitsignal zu bestimmen
- die Abtasteinheit und die Nachbarabtasteinheit mit der Auswertungseinheit für eine simultane Erfassung eines Laufzeitprofils aus dem Laufzeitsignal und eines Nachbarlaufzeitprofils aus dem Nachbarlaufzeitsignal ausgebildet und verbunden sind, und
- die Auswertungseinheit ausgebildet ist, um das Laufzeitprofil und das Nachbarlaufzeitprofil unter Berücksichtigung des Abstands und einer Verfahrgeschwindigkeit miteinander zu vergleichen und um Laufzeitsignale, die sich nicht in dem Laufzeitprofil und dem Nachbarlaufzeitprofil übereinstimmend auffinden, als Lebendsignale zu bewerten.

Gemäß dieser Ausführungsform sind zwei Abtasteinheiten, nämlich die Abtasteinheit und die Nachbarabtasteinheit vorgesehen, die solcherart zueinander angeordnet sind, dass sie in Verfahrrichtung zueinander fluchtende Abtastvorrichtungen haben und daher versetzte aber übereinstimmende Bereiche durch die Verfahrbewegung abtasten. Die Abtastvorrichtung der Abtasteinheit und der Nachbarabtasteinheit sind daher beispielsweise auf gleicher Höhe angeordnet, also insbesondere nur horizontal zueinander versetzt, wenn eine horizontal ausgerichtete Verfahrbewegung während der Messung stattfindet. Die beiden Abtasteinheiten erstellen daher übereinstimmende und lediglich horizontal versetzte Laufzeitprofilmuster, die durch die Auswertungseinheit durch einen entsprechenden rechnerischen Versatz in Übereinstimmung für einen Vergleich gebracht werden können. Die Abtasteinheit und die Nachbarabtasteinheit führen die Messung dabei simultan durch, sodass ein bestimmter Ort innerhalb der Tierhaltungseinrichtung zunächst von einer der beiden Abtasteinheiten und dann von der anderen der beiden Abtasteinheiten abgetastet wird, mit einem entsprechenden zeitlichen Intervall, das sich aus dem Abstand zwischen Abtasteinheit und Nachbarabtasteinheit und der Verfahrgeschwindigkeit ergibt.

Diese spezielle Anordnung und Weiterbildung ermöglicht es, in einem einzigen Überwachungsmessvorgang bereits einen oftmals zuverlässigen Hinweis auf sich bewegende und sich nicht bewegende Tiere zu erhalten, insbesondere werden Tiere durch die Verfahrbewegung der Abtasteinrichtung zu einer Bewegung veranlasst, beispielsweise, weil diese Verfahrbewegung das Interesse der Tiere weckt oder eine Futtergabe Erwartungshaltung auslöst, wenn die Verfahrbewegung an einem Futterspender stattfindet. Es wird somit direkt ermöglicht, kranke oder tote Tiere zu identifizieren, da diese in dem Laufzeitprofil der Abtasteinheit und dem Laufzeitprofil der Nachbarabtasteinheit übereinstimmende Laufzeitsignale erzeugen, wenn sie stationär in der Tierhaltungseinrichtung unbeweglich sich befinden. Die Fortbildung ermöglicht daher eine schnelle Erkennung solcher kranker oder toter Tiere.

Noch weiter ist es bevorzugt, dass die Nachbarabtasteinheit in einer Nachbarabtastvorrichtung sendet und empfängt, die nicht-parallel zu der Abtastvorrichtung der Abtasteinheit verläuft und die in einer planen Ebene mit der Abtastvorrichtung und der Verfahrrichtung liegt, wobei die Nachbarabtastvorrichtung vorzugsweise solcherart ausgerichtet ist, dass sie in einer Referenzebene, die senkrecht zur Verfahrrichtung ausgerichtet ist, in einem Winkel zur Vertikalen steht, der übereinstimmend mit dem Winkel ist, in dem die Abtasteinrichtung in dieser Referenzebene zur Vertikalen steht, und dass die Auswertungseinheit ausgebildet ist, um anhand des Laufzeitsignals, des Nachbarlaufzeitsignals, des Abstands zwischen der Abtasteinheit und der Nachbarabtasteinheit und des Winkels zwischen der Abtastvorrichtung und der Nachbarabtastvorrichtung das Laufzeitprofil und das Nachbarlaufzeitprofil unter Berücksichtigung des Abstands und der Verfahrgeschwindigkeit miteinander zu vergleichen und um Laufzeitsignale, die sich nicht in dem Laufzeitprofil und dem Nachbarlaufzeitprofil übereinstimmend auffinden, als Lebendsignale zu bewerten. Gemäß dieser Fortbildung der Ausgestaltung mit Abtasteinheit und Nachbarabtasteinheit verlaufen die Abtastvorrichtungen der Abtasteinheit und der Nachbarabtasteinheit nicht parallel zueinander, sondern sind in einem Winkel zueinander. Die beiden Abtastvorrichtungen definieren hierbei eine Messebene, in der die beiden Abtastvorrichtungen liegen, die typischerweise horizontal oder unter einem kleinen Winkel zur Horizontalen geneigt liegt und in der ebenfalls die Verfahrrichtung liegt. Durch die Winkellage zwischen der Abtastvorrichtung der Abtasteinheit und der Abtastvorrichtung der Nachbarabtasteinheit können Schattenwirkungen, die durch eine Verdeckung von Objekten durch dazwischenliegende Objekte erzeugt werden, kompensiert werden und hierdurch eine insgesamt bessere Erfassung des gesamten Tierhaltungsbereichs erreicht werden. Um bei dieser Abtastweise die Laufzeitsignale, die mit der Abtasteinheit und die Laufzeitsignale, die mit der Nachbarabtasteinheit gewonnen werden, einem übereinstimmenden Objekt, das abgetastet wird, zuzuordnen, ist die Auswertungseinheit ausgebildet, um anhand der geometrischen Verhältnisse, also dem Abstand zwischen Abtasteinheit und Nachbarabtasteinheit, der Winkelausrichtung der beiden Abtastvorrichtungen und dem jeweiligen Laufzeitsignal die Auswertung vorzunehmen und hierdurch zu ermitteln, ob mit der Abtasteinheit und der Nachbarabtasteinheit ein übereinstimmendes Objekt erfasst wurde. Dies ermöglicht eine Vermeidung von Schattenwirkungen bei gleichzeitiger schneller Erfassung nicht bewegter Objekte in einem einzigen Messvorgang.

Noch weiter ist es bevorzugt, wenn die elektromagnetische Strahlung eine Laserstrahlung ist. Durch eine Laserstrahlung als elektromagnetische Strahlung wird eine zuverlässige und klar ausgerichtete Strahlung in genau einer Richtung eingesetzt, um die Abtastung durchzuführen. Die Laserstrahlung kann dabei in einem sichtbaren oder unsichtbaren Bereich sein, wobei sich diese Angaben sowohl auf die Sichtbarkeit für das menschliche Auge als auch auf die Sichtbarkeit für das jeweilige Sehempfinden eines in der Tierhaltungseinrichtung gehaltenen Tieres beziehen können.

Noch weiter ist es bevorzugt, dass die elektromagnetische Strahlung eine Wellenlänge in einem für ein Huhn nicht sichtbaren Wellenlängenbereich aufweist. Durch die Auswahl eines solchen Wellenlängenbereichs wird die Störung der in der Tierhaltungseinrichtung gehaltenen Hühner durch den Überwachungsvorgang verringert und die Gefahr von Sehkraftbeeinträchtigungen vermieden.

Noch weiter ist es bevorzugt, wenn jede Abtasteinheit einen eindimensionalen und in seiner Winkelausrichtung konstanten elektromagnetischen Strahl aussendet. Eine solche Ausgestaltung der Abtasteinheit ist insbesondere kostengünstig robust und zuverlässig und ermöglicht die zuvor genannten Ausführungsformen mit vertikal gestaffelten mehreren Abtasteinheiten und gegebenenfalls jeweils vorgesehenen Nachbarabtasteinheiten zu jeder der Abtasteinheiten mit einer wirtschaftlichen Investition und einem wirtschaftlichen Wartungsaufwand sowie einer durch die Auswertungseinheit schnell und zuverlässig zu verarbeitenden Datenmenge.

Noch weiter ist es bevorzugt, wenn jede Abtasteinheit einen gepulsten elektromagnetischen Strahl aussendet. Durch eine Abgabe von gepulsten elektromagnetischen Strahlen, also einer regelmäßig oder unregelmäßig unterbrochenen Strahlung, kann eine zuverlässige Laufzeitmessung durchgeführt werden und hierdurch die Auswertung vereinfacht werden. Neben der gepulsten Strahlung sind auch andere Modulationsverfahren denkbar, beispielsweise eine Frequenz- oder Amplitudenmodulation, um hierdurch bei einer konstant ausgesandten Strahlung eine Laufzeitmessung durchführen zu können.

Gemäß einer weiteren bevorzugten Ausführungsform kann die erfindungsgemäße Erfassungsvorrichtung fortgebildet werden durch einen berührungslos messenden Temperatursensor, der für eine Temperaturmessung entlang der Abtastvorrichtung und benachbart oder koaxial zur Abtastvorrichtung angeordnet ist. Ein solcher Temperatursensor ermöglicht die Erfassung eines zusätzlichen Messparameters neben der Ortsbestimmung und der aus dieser Ortsbestimmung abgeleiteten Bewegungsbestimmung. Mit dem Temperatursensor kann unmittelbar als absolute Größe eine Körpertemperatur eines erfassten Tieres bestimmt werden und aufgrund dieser Körpertemperatur auf eine Erkrankung oder auf ein totes Tier geschlossen werden. Dabei kann einerseits der Rückschluss auf eine Erkrankung oder einen Tod des Tieres alleinig auf die absolut gemessene Temperatur gestützt werden oder auf einen Vergleich von zwei Temperaturen, die in einem zeitlichen Abstand durch den Temperatursensor für ein und dasselbe Objekt ermittelt wurden. Die Zuordnung eines Objekts als ein und demselben Objekt erfolgt dabei anhand der Abtastung und der Ortszuordnung des Objekts, sodass mit dem Temperatursensor in zuverlässigerer Weise zwischen einem ruhenden Tier und einem toten Tier unterschieden werden kann, wenn das Tier beispielsweise in einem kürzeren zeitlichen Abstand an ein und demselben Ort erfasst wird. Weiterhin kann mithilfe des Temperatursensors eine Differenzierung zwischen hinzugekommenen Objekten, wie beispielsweise gelegten Eiern oder Kotanhäufungen und ruhenden Tieren erfolgen, da solche Objekte eine andere Temperatur und/oder eine andere Temperaturänderungsrate aufweisen als ein Tier.

Dabei ist es besonders bevorzugt, wenn der Temperatursensor mit der Auswertungseinheit signaltechnisch verbunden ist und die Auswertungseinheit ausgebildet ist, um ein mit der Abtastvorrichtung erfasstes Signal und ein zeitgleich mit dem Temperatursensor erfasstes Signal einander zuzuordnen. Mit dieser speziellen Verbindung der Laufzeitsignale und der Temperaturmesssignale wird eine direkte Zuordnung von den Messdaten, die an einem Objekt bei einem Messdurchgang gewonnen werden, möglich und hierdurch einerseits ein guter Vergleich von zwei Messdurchgängen eröffnet und eine direkte Auswertung eines einzigen Messdurchgangs anhand des dabei ermittelten Laufzeitprofils und der den im Laufzeitprofil erkennbaren Objekte zugeordneten Temperatur möglich, woraus eine direkte Erkenntnis über erkrankte Tiere und tote Tiere möglich wird, beispielsweise indem ein Tier, das nicht auf seinen Beinen steht und eine gegenüber der Normaltemperatur abgesenkte Körpertemperatur aufweist, als ein totes Tier erkannt wird.

Erfindungsgemäss ist vorgesehen, dass die Auswertungseinheit eine Zähleinrichtung umfasst und ausgebildet ist, um in der Zähleinrichtung die Anzahl von in zeitlich regelmäßigem Abstand und/oder in räumlich regelmäßiger Beabstandung von der Abtasteinrichtung erfassten Strukturen zu speichern. Durch diese Fortbildung werden regelmäßig angeordnete Strukturen, wie beispielsweise Vertikalstreben des Geflügelstalls oder sonstige Einrichtungskomponenten erfasst und deren Anzahl fortlaufend gezählt und abgespeichert. Anhand der jeweils an einem bestimmten Abtastort bzw. zu einem bestimmten Abtastzeitpunkt gezählte Anzahl solcher Strukturen kann die Auswertungseinheit eine direkte Ortsbestimmung durchführen, sodass die räumliche Lage eines erfassten störenden Objekts oder eines toten Tieres im Stall direkt aus dieser ermittelten Anzahl bestimmt und zugeordnet werden kann. So kann die Auswertungseinheit als Strukturen, die bei dem Abtastdurchlauf erfasst werden, also als wiederkehrende Strukturen, die Vertikalstreben eines Geflügelstalls erfassen und zählen, sodass anhand der Anzahl der bis zu einem unerwünschten Objekt oder einem toten Tier gezählten Vertikalstreben ein genauer Rückschluss auf die räumliche Position des unerwünschten Objekts oder des toten Tieres erreicht wird.

Gemäß einer weiteren bevorzugten Ausführungsform ist vorgesehen, dass die Auswertungseinheit ausgebildet ist, um anhand des durch die Zähleinrichtung ermittelten zeitlich regelmäßigen Abstands und/oder der durch die Zähleinrichtung ermittelten räumlich regelmäßigen Beabstandung der von der Abtasteinrichtung erfassten Strukturen, eine Position der Abtastteinrichtung entlang eines Verfahrwegs zu bestimmen und diese Position dem Laufzeitprofil zuzuordnen. Gemäß dieser Ausführungsform wird die Zähleinrichtung dazu eingesetzt, um eine präzise Positionsbestimmung der Abtasteinrichtung durchzuführen und dem Laufzeitprofil zuzuordnen. Dabei ist zu verstehen, dass vorteilhafterweise vor der Messung eines Laufzeitprofils in einem mit Tieren besetzten Stall ein Kalibrierungsvorgang durchgeführt wird, der anhand eines unbesetzten Stalles eine Identifikation und nachfolgende zweifelsfreie Zuordnung von wiederkehrenden Strukturen, wie beispielsweise Vertikalstreben oder sonstigen Einbauten erlaubt.

Die Bestimmung einer bestimmten Position anhand der ermittelten wiederkehrenden Strukturen kann dabei einerseits solcherart erfolgen, dass dem Benutzer durch die Auswertungseinheit eine Position angegeben wird, die durch eine im Laufweg der Abtasteinrichtung vorhergehende und nachfolgende wiederkehrende Struktur angegeben wird, beispielsweise indem eine Angabe solcherart erfolgt, dass die bestimmte Position zwischen der fünften und sechsten wiederkehrenden Struktur (Vertikalstrebe) im Laufweg der Abtasteinheit liegt.

Die Positionsangabe kann weiter präzisiert werden, indem die Auswertungseinheit ausgebildet ist, um anhand der verstrichenen Laufzeit der Abtasteinrichtung während des Verfahrens entlang des Verfahrwegs eine Interpolation zwischen zwei wiederkehrenden Strukturen erfolgt und anhand dieser Interpolation die exakte Position der Abtasteinrichtung im Bereich zwischen den beiden wiederkehrenden Strukturen berechnet wird. So kann beispielsweise dann, wenn zwischen der Erfassung von zwei aufeinanderfolgenden wiederkehrenden Strukturen ein Zeitraum X verstrichen ist, und eine bestimmte Position erreicht wurde, nachdem die Abtasteinrichtung über einen Zeitraum von 80 % von X nach Erfassen der ersten wiederkehrenden Struktur erreicht wurde und zugleich - gegebenenfalls - als Kontrollmöglichkeit die darauffolgende wiederkehrende Struktur, ausgehend von der Position, nach 20 % der Verfahrzeit erreicht wurde, geschlossen werden, dass die gesuchte Position bei 80 % der Gesamtdistanz zwischen den beiden wiederkehrenden Strukturen hinter der ersten wiederkehrenden Struktur entfernt ist und entsprechend 20 % der Distanz vor der zweiten wiederkehrenden Struktur gelegen ist. Auf diese Weise kann die Auswertungseinheit ausgebildet sein, anhand des Zeitablaufs zwischen der Erfassung von zwei aufeinanderfolgenden wiederkehrenden Strukturen eine präzise Position zwischen den beiden wiederkehrenden Strukturen anzugeben und so das Auffinden einer gesuchten, im Laufzeitprofil erkannten Struktur erleichtern.

Noch weiter ist es bevorzugt, wenn die Tierüberwachungsvorrichtung fortgebildet wird durch einen mit einem durch eine Antriebseinheit angetriebenen oder mit einem passiv angetriebenen Übertragungselement, insbesondere einem Antriebsrad oder einem Reibrad gekoppelten, Sensor zur Erfassung der Wegdistanz des Übertragungselements, wobei die Auswertungseinheit mit dem Sensor signaltechnisch gekoppelt und ausgebildet ist, um aus einem von dem Sensor empfangenen Wegdistanzsignal eine Position der Abtastteinrichtung entlang eines Verfahrwegs zu bestimmen und diese Position dem Laufzeitprofil zuzuordnen. Gemäß dieser Ausführungsform ist ein Sensor vorgesehen, beispielsweise ein Odometer, Inkrementalgeber, Drehsensor, welcher die zurückgelegte Strecke der Abtasteinrichtung erfasst. Dieser Sensor kann einerseits direkt mit einem Rad mechanisch gekoppelt sein, das zum Antrieb der Abtasteinrichtung entlang der zu messenden Strecke dient. Der Sensor kann aber auch mit einem passiv abrollenden Rad gekoppelt sein, beispielsweise einem Reibrad, das an einer Struktur abrollt, die sich längs des Verfahrwegs erstreckt. In beiden Fällen wird eine Erfassung des zurückgelegten Wegs anhand der Sensorsignale ermöglicht und kann mit dem Laufzeitprofil kombiniert werden, um eine Positionsangabe für einen gesuchten Punkt in dem Laufzeitprofil für den Benutzer auffindbar zu machen.

Dabei ist zu verstehen, dass diese Fortbildung mit einem Sensor zur Positionsbestimmung insbesondere auch kombiniert werden kann mit einer Positionsbestimmung nach einer der zuvor erläuterten Fortbildungsformen. Insbesondere kann diese sensorgestützte Positionsbestimmung mit einer Positionsbestimmung anhand der Erfassung von wiederkehrenden Strukturen durch die Abtasteinrichtung selbst kombiniert werden, um hierdurch eine Verifizierung der Positionsangabe durchzuführen. Die Auswertungseinheit kann insoweit dazu ausgebildet sein, um diese zwei unterschiedlichen Positionsbestimmungsweisen miteinander zu kombinieren und hieraus eine Positionsangabe oder eine Positionsbereichsangabe zu erzeugen. Grundsätzlich können neben diesen Möglichkeiten einer Positionsbestimmung auch weitere Positionsbestimmungsvorrichtungen zusätzlich oder alternativ vorgesehen sein, um innerhalb des Laufzeitprofils einer gesuchten Struktur eine Position zuzuordnen und hierdurch das Auffinden der Struktur dem Benutzer zu erleichtern. So kann beispielsweise die Abtasteinrichtung selbst so ausgebildet sein, dass sie in regelmäßigen oder unregelmäßigen zeitlichen Abständen eine Abtastung in Verfahrrichtung ausführt, also vorwärts- oder rückwärtsgerichtet. Anhand dieser Abtastung in Verfahrrichtung, die gegebenenfalls auf eine bestimmte Reflexstruktur am Anfang oder am Ende des Verfahrweges der Abtasteinrichtung gerichtet ist und von dieser Reflexstruktur reflektiert wird, kann dann eine Positionsbestimmung entlang des Verfahrwegs erfolgen und dem Zeitpunkt bzw. dem Signalwert des Laufzeitprofils zugeordnet werden, das kurz vor oder kurz nach dieser Abtastung in Verfahrrichtung aufgenommen wurde. Durch wiederholte Abtastung in Verfahrrrichtung können hierdurch mehrere Positionen entlang des Verfahrwegs zeitlich dem Laufzeitprofil zugeordnet werden und hierdurch eine Positionsbestimmung von gesuchten Strukturen innerhalb des Laufzeitprofils ermöglicht werden. Auch hier kann wiederum durch Interpolation zwischen zwei Positionsbestimmungen anhand dieser Abtastung in Verfahrrichtung eine exakte Positionsbestimmung für jeden Ort entlang des Laufzeitprofils mittels der Auswertungseinheit erfolgen.

Grundsätzlich ist zu verstehen, dass auf Grundlage dieser Positionsbestimmungen, welche mittels der Erfassung wiederkehrender Strukturen, mittels eines separaten Sensors oder mittels wiederholter Abtastung in Verfahrrichtung erfolgt, das Laufzeitprofil in ein Ortsprofil überführt werden, sodass die Auswertungseinheit den einzelnen Laufzeitsignalen, die entlang des Verfahrwegs aufgenommen wurden, jeweils anstelle eines Signalerfassungszeitpunktes einen Ortserfassungspunkt zuordnet, welcher die Position entlang des Verfahrwegs charakterisiert, an der das Laufzeitsignal erfasst wurde. Durch diese Überführung des Laufzeitprofils in ein Ortsprofil wird die einfache Aufwendung einer bestimmten, anhand der Laufzeitsignale erkennbaren Struktur schnell und sicher möglich. Eine bevorzugte Ausführungsform der Erfindung wird anhand der beiliegenden Figuren erläutert. Es zeigen:
- Fig. 1: eine schematische Längsansicht einer erfindungsgemäßen Erfassungsvorrichtung im Einsatz an einer Tierhaltungseinrichtung für Hühner mit drei Etagen,
- Fig. 2: einen Ausschnitt aus Fig. 1, der die Messanordnung verdeutlicht, in einer perspektivischen Anordnung,
- Fig. 3: ein Laufzeitprofil, und
- Fig. 4: ein Laufzeitprofilmuster.

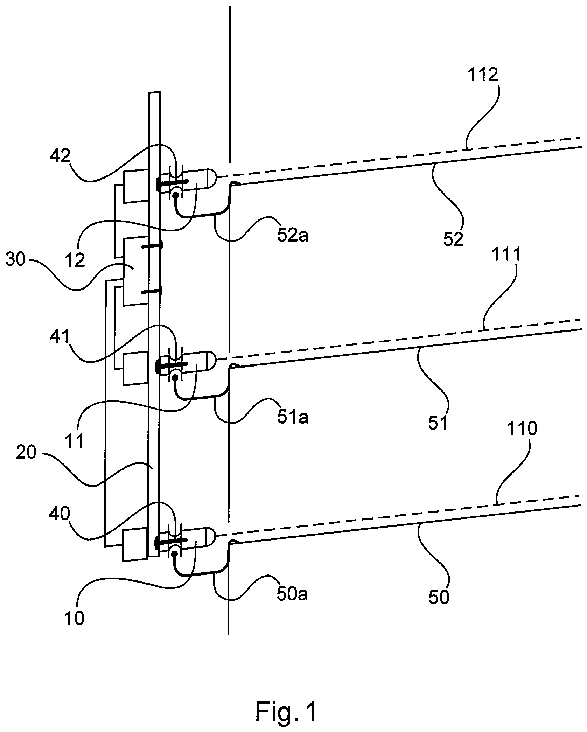

Wie aus Fig. 1 ersichtlich, ist die erfindungsgemäße Erfassungsvorrichtung mit einer Abtasteinrichtung ausgerüstet, die drei vertikal zueinander beabstandete Abtasteinheiten 10, 11, 12 umfasst. Die Abtasteinheiten 10, 11, 12 sind an einem Gestell 20 befestigt, an dem auch eine Auswertungseinheit 30 angeordnet ist, mit der die Abtasteinheiten 10, 11, 12 signaltechnisch verbunden sind.

Das Rahmengestell 20 ist mittels drei Rollen 40, 41, 42 auf den Eiersammelrinnen 50a, 51a, 52a der Käfigeinrichtung beweglich gelagert und können somit in einer Verfahrrichtung A entlang der Käfige bewegt werden.

Jede der Abtasteinheiten sendet einen Laserstrahl 110, 111, 112 aus, der in geringer Beabstandung oberhalb einer Bodenaufstandsfläche 50, 51, 52 des Käfigs verläuft.

Die Abtastung erfolgt vorzugsweise in einer Ebene oberhalb der Aufstandsfläche der Tiere, die von der Aufstandsfläche 0,5 bis 10cm, vorzugsweise 1-5cm beanstandet ist. Der Abstand ist solcherart zu wählen, dass auf Höhe der Beine der Tiere die Abtastung erfolgt. Dies sichert eine differenzierte Abtastung und Erkennung der Tiere und Objekte.

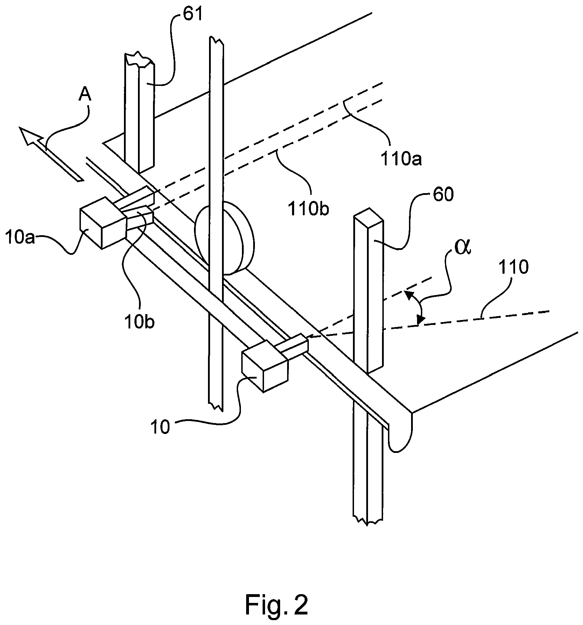

In Verfahrrichtung A horizontal voneinander beabstandet ist jeder Abtasteinheit eine Nachbarabtasteinheit zugeordnet, wie in Fig. 2 anhand der Abtasteinheit 10 und der Nachbarabtasteinheit 10a ersichtlich. Die Abtasteinheit und die Nachbarabtasteinheit messen beide mit einem Laserstrahl, der parallel in einem geringen Abstand oberhalb der Bodenfläche 40 verläuft. Der Lasermessstrahl 110 der Abtasteinheit steht jedoch in einem Winkel α zum Laserstrahl 110a der Nachbarabtasteinheit 10a, sodass Verschattungen, die sich durch Abdeckung von Objekten durch Objekte ergeben, die zwischen der Abtasteinheit und dem dahinterliegenden Objekt ergeben, vermieden werden können und alle Objekte im Bereich oberhalb der Bodenfläche 40 durch zumindest einen der beiden Laserstrahlen 110, 110a erfasst werden.

Der Nachbarerfassungseinheit 10a ist eine direkt daran befestigte berührungslos messende Temperaturmesseinheit 10b zugeordnet, die einen Temperaturmessstrahl 110b aussendet, der parallel zum Lasermessstrahl 110a verläuft.

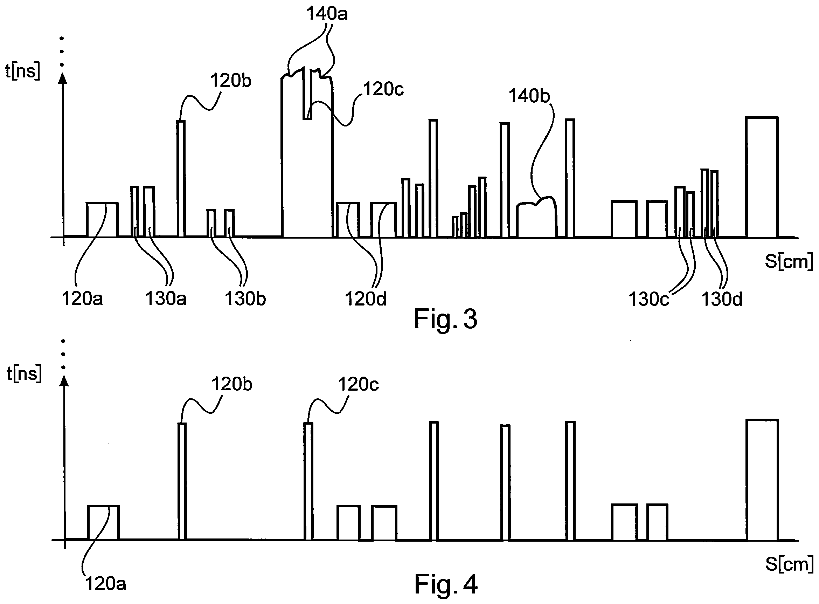

Fig. 3 zeigt ein typisches Laufzeitprofil für eine Messung, die mit einer Abtasteinheit durchgeführt wurde. Auf der X-Achse des Laufzeitprofils ist der Verfahrweg entlang der Eiersammelrinne aufgetragen, auf der Y-Achse ist das Laufzeitsignal aufgetragen, das jeweils mit dem Lasermessstrahl 110 an dem entsprechenden Ort entlang der Eiersammelrinne ermittelt wurde. Fig. 34 zeigt ein entsprechendes Laufzeitprofilmuster, das durch eine Kalibrierungsfahrt aufgenommen wurde, bei der keine Tiere in der Tierhaltungseinrichtung vorhanden waren. Wie im Abgleich zwischen der Fig. 3 und 4 gut erkennbar, sind wiederkehrende Laufzeitsignale 120a, b, c, d direkt einem ortsfesten Element der Stalleinrichtung zuzuordnen, wie beispielsweise vertikalstehenden Streben 60, 61, Tränkeinrichtungen oder dergleichen. Anhand des Musters der so verteilten Laufzeitsignale im Laufzeitprofilmuster und diesen zuzuordnenden Laufzeitsignalen im Laufzeitprofil ist eine zuverlässige Ortsangabe für jedes Laufzeitsignal, das sich zusätzlich im Laufzeitprofil befindet, zu ermitteln.

Im Laufzeitprofil finden sich dann weiterhin typische Laufzeitsignale 130a, a, c, d, die für Beine von stehenden Hühnern ermittelt werden, dies sind schmale Peaks im Laufzeitsignal, die durch die Reflexion des Laserstrahls an diesen Beinen entstehen.

Ein auf der Bodenfläche 50 liegendes breites Objekt oder Huhn ist durch eine breite Laufzeitsignalprofilerhebung 140a, in dem Laufzeitprofil erkennbar. Wird eine solche breite Laufzeitprofilerhebung 140a, wiederholt an genau dem gleichen Ort festgestellt, muss hieraus auf ein stationäres Objekt oder ein totes oder krankes Huhn geschlossen werden, das sich zwischen den beiden Messungen nicht bewegt hat.

Neben der Möglichkeit, ein solches stationäres, den Eierabrollprozess störendes Objekt oder ein vermutet krankes oder totes Tier durch zwei zeitlich beabstandete Laufzeitprofilerhebungen mit unveränderter räumlicher Anordnung festzustellen, kann auch aus einer einmalig festgestellten breiten Laufzeitprofilerhebung, die auf ein am Boden liegendes Objekt oder Tier schließen lässt, gepaart mit einer Temperaturmessung, die auf eine für ein lebendes Huhn untypische Temperatur des Objekts bzw. auf eine abgesenkte Körpertemperatur des Tieres hinweist, auf ein entsprechend stationäres Objekt bzw. krankes oder vermutlich totes Tier geschlossen werden.

## Patentansprüche

1. Tierüberwachungsvorrichtung zur Erfassung von stationären Objekten in einem Geflügelstall, umfassend:
eine berührungslos arbeitende Abtastvorrichtung, die ausgebildet ist, um eine elektromagnetische Strahlung in einer Abtastrichtung auszusenden und eine Reflexion der elektromagnetischen Strahlung zu empfangen,
eine mit der Abtastvorrichtung signaltechnisch verbundene elektronische Auswertungseinheit (30), die ausgebildet ist, um die von der Abtastvorrichtung empfangenen Signale auszuwerten,
wobei
die Abtastvorrichtung mindestens eine Abtasteinheit (10, 11, 12) umfasst, die einen Sender zum Aussenden eines elektromagnetischen Abtaststrahls (110, 111, 112) in einer vorbestimmten Richtung und einen Empfänger zum Empfang einer Reflexion des Abtaststrahls aus der vorbestimmten Richtung umfasst und die Auswertungseinheit (30) ausgebildet ist, um aus dem Empfang eines von der Abtasteinheit (10, 11, 12) ausgesendetem und reflektierten Signals und dessen Laufzeit ein Laufzeitsignal (120a-d, 130a-d) zu bestimmen und um aus dem über einen Zeitraum empfangenen Laufzeitsignal ein Laufzeitprofil für die Abtasteinheit zu erstellen,
**dadurch gekennzeichnet, dass**
die Auswertungseinheit (30) ausgebildet ist, um
- In einem Kalibrierungsmodus ein erstes Laufzeitprofil aufzunehmen und das erste Laufzeitprofil als Laufzeitprofilmuster in dem Laufzeitprofilspeicher abzuspeichern, und
- In einem Überwachungsmodus ein zweites Laufzeitprofil und nachfolgend gegebenenfalls weitere Laufzeitprofile aufzunehmen und dieses zweite bzw. jedes weitere Laufzeitprofil mit dem Laufzeitprofilmuster zu vergleichen,
wobei die Auswertungseinheit (30) eine Zähleinrichtung umfasst und ausgebildet ist, um in der Zähleinrichtung die Anzahl von in zeitlich regelmäßigem Abstand und/oder in räumlich regelmäßiger Beabstandung von der Abtasteinrichtung erfassten Strukturen zu speichern.

2. Tierüberwachungsvorrichtung nach Anspruch 1,
**dadurch gekennzeichnet, dass** die Abtastvorrichtung mindestens zwei vertikal beabstandete Abtasteinheiten (10, 11, 12) umfasst, von denen jede Abtasteinheit einen Sender zum Aussenden eines elektromagnetischen Abtaststrahls (110, 111, 112) in einer vorbestimmten Richtung und einen Empfänger zum Empfang einer Reflexion des Abtaststrahls aus der vorbestimmten Richtung umfasst, und die Auswertungseinheit (30) ausgebildet ist, um aus dem Empfang eines von jeder Abtasteinheit ausgesendetem und reflektierten Signals und dessen Laufzeit ein Laufzeitsignal (120a-d, 130a-d) zu bestimmen und um aus den über einen Zeitraum empfangenen Laufzeitsignalen ein Laufzeitprofil für jede der Abtasteinheiten zu erstellen.

3. Tierüberwachungsvorrichtung nach Anspruch 1,
**dadurch gekennzeichnet, dass** die Auswertungseinheit (30) einen elektronischen Laufzeitprofilspeicher umfasst und weiterhin dazu ausgebildet ist, um
- In dem Laufzeitprofilspeicher mindestens ein vorbestimmtes Laufzeitprofilmuster und einer diesem vorbestimmten Laufzeitprofilmuster zugeordnete Ortsangabe abzuspeichern,
- aus einer Mehrzahl von über einen Abtastzeitraum ermittelten Laufzeitsignalen ein Laufzeitprofil zu erstellen, welches die Laufzeitsignale (120a-d, 130a-d) während des Abtastzeitraums darstellt,
- mindestens einen Ausschnitt des Laufzeitprofils mit dem in dem Laufzeitprofilspeicher gespeicherten vorbestimmten Laufzeitprofilmuster zu vergleichen, und
- bei festgestellter Übereinstimmung des mindestens einen Ausschnitts des Laufzeitprofils mit dem Laufzeitprofilmuster dem Ausschnitts aus dem Laufzeitprofil die in Laufzeitprofilspeicher zu dem vorbestimmten Laufzeitprofilmuster gespeicherte Ortsangabe zuzuordnen.

4. Tierüberwachungsvorrichtung nach Anspruch 3,
**dadurch gekennzeichnet, dass** die Auswertungseinheit (30) ausgebildet ist, um
- bei dem Vergleich des mindestens einen Ausschnitt des Laufzeitprofils mit dem in dem Laufzeitprofilspeicher gespeicherten vorbestimmten Laufzeitprofilmuster einen ersten Laufzeitprofilabschnitt, einen zweiten Laufzeitprofilabschnitt und einen Ortsabstand, der eine räumliche Distanz zwischen dem ersten Laufzeitprofilabschnitt und dem zweiten Laufzeitprofilabschnitt definiert, mit einem ersten Laufzeitprofilmusterabschnitt des Laufzeitprofilmusters, einem zweiten Laufzeitprofilmusterabschnitt des Laufzeitprofilmusters und einem vorbestimmten Ortsabstand, der eine Distanz zwischen dem ersten Laufzeitprofilmusterabschnitt und dem zweiten Laufzeitprofilmusterabschnitt definiert, zu vergleichen, und
- bei festgestellter Übereinstimmung des ersten Laufzeitprofilabschnitts mit dem ersten Laufzeitprofilmusterabschnitt und festgestellter Übereinstimmung des zweiten Laufzeitprofilabschnitts mit dem zweiten Laufzeitprofilmusterabschnitt und festgestellter Übereinstimmung des Ortsabstands mit dem vorbestimmten Ortsabstand dem Laufzeitprofil die in dem Laufzeitprofilspeicher zu dem vorbestimmten Laufzeitprofilmuster gespeicherte Ortsangabe zuzuordnen.

5. Tierüberwachungsvorrichtung nach einem der vorhergehenden Ansprüche,
**dadurch gekennzeichnet, dass** die elektromagnetische Strahlung eine Laserstrahlung ist.

6. Tierüberwachungsvorrichtung nach einem der vorhergehenden Ansprüche,
**gekennzeichnet durch** eine Verfahreinrichtung zum Verfahren der Abtastvorrichtung entlang einer Tierhaltungseinrichtung in einer Verfahrrichtung (A), die quer, insbesondere senkrecht zur Abtastvorrichtung ausgerichtet ist.

7. Tierüberwachungsvorrichtung nach Anspruch 6,
**dadurch gekennzeichnet, dass** die Auswertungseinheit (30) ausgebildet ist, um ein Laufzeitprofil während einer kontinuierlichen Bewegung der Abtasteinrichtung entlang der Tierhaltungseinrichtung zu erfassen, wobei die Tierhaltungseinrichtung vorzugsweise eine Mehrzahl von separaten, voneinander abgetrennten Tierhaltungseinheiten aufweist, und die Auswertungseinheit ausgebildet ist, um die Mehrzahl der separaten, voneinander abgetrennten Tierhaltungseinheiten während der Verfahrbewegung in einem einzigen Laufzeitprofil zu erfassen und mit einem für die Mehrzahl der separaten, voneinander abgetrennten Tierhaltungseinheiten abgespeicherten Laufzeitprofilmuster zu vergleichen.

8. Tierüberwachungsvorrichtung nach einem der vorhergehenden Ansprüche 6 oder 7,
**dadurch gekennzeichnet, dass** in Verfahrrichtung (A) um einen Abstand beabstandet und in Bezug auf die Verfahrrichtung fluchtend zu einer Abtasteinheit eine Nachbarabtasteinheit (10a) angeordnet ist, wobei
- Die Auswertungseinheit (30) ausgebildet ist, um aus dem Empfang eines von der Nachbarabtasteinheit (10a) ausgesendetem Signals und dessen Laufzeit ein Nachbarlaufzeitsignal zu bestimmen
- die Abtasteinheit und die Nachbarabtasteinheit mit der Auswertungseinheit für eine simultane Erfassung eines Laufzeitprofils aus dem Laufzeitsignal (120a-d, 130a-d) und eines Nachbarlaufzeitprofils aus dem Nachbarlaufzeitsignal ausgebildet und verbunden sind, und
- die Auswertungseinheit (30) ausgebildet ist, um das Laufzeitprofil und das Nachbarlaufzeitprofil unter Berücksichtigung des Abstands und einer Verfahrgeschwindigkeit miteinander zu vergleichen und um Laufzeitsignale, die sich nicht in dem Laufzeitprofil und dem Nachbarlaufzeitprofil übereinstimmend auffinden, als Lebendsignale zu bewerten.

9. Tierüberwachungsvorrichtung nach Anspruch 8,
**dadurch gekennzeichnet, dass** die Nachbarabtasteinheit (10a) in einer Nachbarabtastvorrichtung sendet und empfängt, die nicht-parallel zu der Abtastvorrichtung der Abtasteinheit verläuft und die in einer planen Ebene mit der Abtastvorrichtung und der Verfahrrichtung (A) liegt,
wobei die Nachbarabtastvorrichtung vorzugsweise solcherart ausgerichtet ist, dass sie in einer Referenzebene, die senkrecht zur Verfahrrichtung ausgerichtet ist, in einem Winkel zur Vertikalen steht, der übereinstimmend mit dem Winkel ist, in dem die Abtasteinrichtung in dieser Referenzebene zur Vertikalen steht,
und dass die Auswertungseinheit (30) ausgebildet ist, um anhand des Laufzeitsignals, des Nachbarlaufzeitsignals, des Abstands zwischen der Abtasteinheit und der Nachbarabtasteinheit und des Winkels zwischen der Abtastvorrichtung und der Nachbar Abtastvorrichtung das Laufzeitprofil und das Nachbarlaufzeitprofil unter Berücksichtigung des Abstands und der Verfahrgeschwindigkeit miteinander zu vergleichen und um Laufzeitsignale, die sich nicht in dem Laufzeitprofil und dem Nachbarlaufzeitprofil übereinstimmend auffinden, als Lebendsignale zu bewerten.

10. Tierüberwachungsvorrichtung nach einem der vorhergehenden Ansprüche,
**dadurch gekennzeichnet, dass** die elektromagnetische Strahlung eine Wellenlänge in einem für ein Huhn nicht sichtbaren Wellenlängenbereich aufweist.

11. Tierüberwachungsvorrichtung nach einem der vorhergehenden Ansprüche,
**dadurch gekennzeichnet, dass** jede Abtasteinheit (10, 11, 12) einen eindimensionalen und in seiner Winkelausrichtung konstanten elektromagnetischen Strahl aussendet.

12. Tierüberwachungsvorrichtung nach einem der vorhergehenden Ansprüche,
**dadurch gekennzeichnet, dass** jede Abtasteinheit (10, 11, 12) einen gepulsten elektromagnetischen Strahl aussendet.

13. Tierüberwachungsvorrichtung nach einem der vorhergehenden Ansprüche,
**gekennzeichnet durch** einen berührungslos messenden Temperatursensor, der für eine Temperaturmessung entlang der Abtastvorrichtung und benachbart oder koaxial zur Abtastvorrichtung angeordnet ist.

14. Tierüberwachungsvorrichtung nach Anspruch 13,
**dadurch gekennzeichnet, dass** der Temperatursensor mit der Auswertungseinheit (30) signaltechnisch verbunden ist und die Auswertungseinheit ausgebildet ist, um ein mit der Abtastvorrichtung erfasstes Signal und ein zeitgleich mit dem Temperatursensor erfasstes Signal einander zuzuordnen.

15. Tierüberwachungsvorrichtung nach Anspruch 1,
**dadurch gekennzeichnet, dass** die Auswertungseinheit (30) ausgebildet ist, um anhand des durch die Zähleinrichtung ermittelten zeitlich regelmäßigem Abstand und/oder der durch die Zähleinrichtung ermittelten räumlich regelmäßigen Beabstandung der von der Abtasteinrichtung erfassten Strukturen eine Position der Abtastteinrichtung entlang eines Verfahrwegs zu bestimmen und diese Position dem Laufzeitprofil zuzuordnen.

16. Tierüberwachungsvorrichtung nach einem der vorhergehenden Ansprüche,
**gekennzeichnet durch** einen mit einem durch eine Antriebseinheit (10, 11, 12) angetriebenen oder mit einem passiv angetriebenen Übertragungselement, insbesondere einem Antriebsrad oder einem Reibrad gekoppelten, Sensor zur Erfassung der Wegdistanz des Übertragungselements, wobei die Auswertungseinheit mit dem Sensor signaltechnisch gekoppelt und ausgebildet ist, um aus einem von dem Sensor empfangenen Wegdistanzsignal eine Position der Abtastteinrichtung entlang eines Verfahrwegs zu bestimmen und diese Position dem Laufzeitprofil zuzuordnen.

## Claims

1. An animal surveillance device for detecting stationary objects in a poultry house, comprising:
a noncontact scanner adapted to emit electromagnetic radiation in a scanning direction and to receive a reflection of the electromagnetic radiation,
an electronic evaluation unit (30) which is in signal communication with the scanner and adapted to evaluate the signals received from the scanner,
wherein the scanner includes at least one scanner unit (10, 11, 12) comprising a transmitter for transmitting an electromagnetic scanning beam (110, 111, 112) in a predetermined direction and a receiver for receiving a reflection of the scanning beam from said predetermined direction and that the evaluation unit (30) is configured to calculate a transit time signal (120a-d, 130a-d) from the reception of a reflected signal emitted from the scanner unit (10, 11, 12) and from the transit time of the signal and to produce a transit time profile for the scanner unit from the transit time signal received over a period,
**characterised in that** the evaluation unit (30) is configured
- to record, in a calibration mode, a first transit time profile and to store the first transit time profile as a transit time profile pattern in the transit time profile memory, and
- to record, in a surveillance mode, a second transit time profile and then any further transit time profiles, if necessary, and to compare said second and each further transit time profile with the transit time profile pattern,
wherein the evaluation unit (30) includes a counting means and is configured to store in the counting means the number of structures detected at regular time intervals and/or at regular spatial intervals by the scanner.

2. The animal surveillance device according to claim 1,
**characterised in that** the scanner includes at least two vertically spaced-apart scanner units (10, 11, 12), each of said scanner units comprising a transmitter for transmitting an electromagnetic scanning beam (110, 111, 112) in a predetermined direction and a receiver for receiving a reflection of the scanning beam from said predetermined direction and that the evaluation unit (30) is configured to calculate a transit time signal (120a-d, 130a-d) from the reception of a reflected signal emitted from the scanner unit and from the transit time of the signal and to produce a transit time profile for each of the scanner units from the transit time signals received over a period.

3. The animal surveillance device according to claim 1,
**characterised in that** the evaluation unit (30) includes an electronic transit time profile memory and is further configured
- to store in the transit time profile memory at least one predetermined transit time profile pattern and a location associated with said predetermined transit time profile pattern,
- to produce, from a plurality of transit time signals detected over a scanning period, a transit time profile representing the transit time signals (120a-d, 130a-d) during said scanning period,
- to compare at least one section of the transit time profile with the predetermined transit time profile pattern stored in the transit time profile memory, and
- to associate, when the at least one section of the transit time profile is found to match the transit time profile pattern, the location stored in the transit time profile memory in respect of the predetermined transit time profile pattern with said section of the transit time profile.

4. The anima! surveillance device according to claim 3,
**characterised in that** the evaluation unit (30) is configured
- to compare, when comparing at least one section of the transit time profile with the predetermined transit time profile pattern stored in the transit time profile memory, a first transit time profile section, a second transit time profile section, and a distance defining a spatial distance between the first transit time profile section and the second transit time profile section, with a first section of the transit time profile pattern, a second section of the transit time profile pattern and a predetermined distance defining a distance between the first second of the transit time profile pattern and the second section of the transit time profile pattern, and
- to associate the location stored in the transit time profile memory in respect of the predetermined transit time profile pattern with the transit time profile when the first transit time profile section is found to match the first section of the transit time profile pattern and the second transit time profile section is found to match the second section of the transit time profile pattern and the distance is found to match the predetermined distance.

5. The animal surveillance device according to any one of the preceding claims,
**characterised in that** the electromagnetic radiation is laser radiation.

6. The animal surveillance device according to any one of the preceding claims,
**characterised by** a traversing device for moving the scanner along an animal housing facility in a traversing direction (A) which is transverse to, in particular perpendicular to the scanner.

7. The animal surveillance device according to claim 6,
**characterised in that** the evaluation unit (30) is configured to record a transit time profile during continuous movement of the scanner along the animal housing facility, wherein the animal housing facility preferably has a plurality of distinct housing units separated from each other, and the evaluation unit is configured to record, in a single transit time profile, during the traversing movement, the plurality of distinct housing units separated from each other and to compare said transit time profile with a transit time profile pattern stored for the plurality of distinct housing units separated from each other.

8. The animal surveillance device according to any one of the preceding claims 6 or 7,
**characterised in that** a neighbouring scanner unit (10a) is arranged flush with a scanner unit in respect of the traversing direction (A) and spaced a distance apart from it in the traversing direction, wherein
- the evaluation unit unit (30) is configured to calculate a neighbouring transit time signal from the reception of a signal emitted from the neighbouring scanner unit (10a) and its transit time,
- the scanner unit and the neighbouring scanner unit with the evaluation unit are configured and interconnected for simultaneous recording of a transit time profile from the transit time signal (120a-d, 130a-d) and of a neighbouring transit time profile from the neighbouring transit time signal, and
- the evaluation unit (30) is configured to compare the transit time profile and the neighbouring transit time profile with each other, taking the spacing and a traversing speed into account, and to evaluate as sign-of-life signals any transit time signals for which no match can be found in the transit time profile and the neighbouring transit time profile.

9. The animal surveillance device according to claim 8,
**characterised in that** the neighbouring scanner unit (10a) transmits and receives in a neighbouring scanner which moves non-parallel to the scanner of the scanner unit and which lies in a level plane with the scanner and the traversing direction (A),
wherein the neighbouring scanner is preferably oriented in such a way that, in a reference plane which is perpendicular to the traversing direction, it is at an angle to the vertical which is the same as the angle at which the scanner is to the vertical in said reference plane,
and **in that** the evaluation unit (30) is configured to compare the transit time profile and the neighbouring transit time profile with each other on the basis of the transit time signal, the neighbouring transit time signal, the spacing between the scanner unit and the neighbouring scanner unit, and the angle between the scanner and the neighbouring scanner, taking the spacing and the traversing speed into account, and to evaluate as sign-of-life signals any transit time signals for which no match can be found in the transit time profile and the neighbouring transit time profile.

10. The animal surveillance device according to any one of the preceding claims,
**characterised in that** the electromagnetic radiation has a wavelength in a range which is not visible to a hen.

11. The animal surveillance device according to any one of the preceding claims,
**characterised in that** each scanner unit (10, 11, 12) emits a one-dimensional electromagnetic beam with a constant angular orientation.

12. The animal surveillance device according to any one of the preceding claims,
**characterised in that** each scanner unit (10, 11, 12) emits a pulsed electromagnetic beam.

13. The animal surveillance device according to any one of the preceding claims,
**characterised by** a contactlessly measuring temperature sensor arranged for temperature measurement along the scanner and adjacent to or coaxially with the scanner.

14. The animal surveillance device according to claim 13,
**characterised in that** the temperature sensor is in signal communication with the evaluation unit (30), and the evaluation unit is configured to associate with one another a signal detected by the scanner and a signal detected simultaneously by the temperature sensor.

15. The animal surveillance device according to claim 1,
**characterised in that** the evaluation unit (30) is configured to calculate a position of the scanner along a traversing path and to associate that position with the transit time profile, on the basis of the regular time interval measured by the counting means and/or the regular spatial interval measured by the counting means between the structures detected by the scanner.

16. The animal surveillance device according to any one of the preceding claims,
**characterised by** a sensor, coupled to a transmission element driven by a drive unit, or to a passively driven transmission element, in particular a drive wheel or a friction wheel, for measuring the distance travelled by the transmission element, wherein the evaluation unit is in signal communication with the sensor and configured to determine, from a distance signal received from the sensor, a position of the scanner along a path of travel and to associate that position with the transit time profile.

## Revendications

1. Dispositif de surveillance d'animaux pour la détection d'objets fixes dans un poulailler, comprenant :
un dispositif de balayage fonctionnant sans contact, qui est réalisé pour envoyer un rayonnement électromagnétique dans une direction de balayage et pour recevoir une réflexion du rayonnement électromagnétique,
une unité d'évaluation (30) électronique reliée par technique de signaux au dispositif de balayage, qui est réalisée pour évaluer les signaux reçus par le dispositif de balayage,
dans lequel le dispositif de balayage comprend au moins une unité de balayage (10, 11, 12), qui comprend un émetteur pour l'émission d'un faisceau de balayage (110, 111, 112) électromagnétique dans une direction prédéfinie et un récepteur pour la réception d'une réflexion du faisceau de balayage provenant de la direction prédéfinie et l'unité d'évaluation (30) est réalisée pour déterminer à partir de la réception d'un signal émis par l'unité de balayage (10, 11, 12) et réfléchi et du temps de propagation de celui-ci un signal de temps de propagation (120a-d, 130a-d) et pour élaborer à partir du signal de temps de propagation reçu pendant une période un profil de temps de propagation pour l'unité de balayage,
**caractérisé en ce que** l'unité d'évaluation (30) est réalisée pour
- dans un mode d'étalonnage enregistrer un premier profil de temps de propagation et mettre en mémoire le premier profil de temps de propagation en tant que modèle de profil de temps de propagation dans la mémoire de profil de temps de propagation, et
- dans un mode de surveillance enregistrer un deuxième profil de temps de propagation et ensuite éventuellement d'autres profils de temps de propagation et comparer ce deuxième ou chaque autre profil de temps de propagation au modèle de profil de temps de propagation,
dans lequel l'unité d'évaluation (30) comprend un système de comptage et est réalisée pour mettre en mémoire dans le système de comptage le nombre de structures détectées par le système de balayage dans un écart régulier dans le temps et/ou dans un espacement régulier dans l'espace.

2. Dispositif de surveillance d'animaux selon la revendication 1,
**caractérisé en ce que** le dispositif de balayage comprend au moins deux unités de balayage (10, 11, 12) espacées verticalement, dont chaque unité de balayage comprend un émetteur pour l'émission d'un faisceau de balayage (110, 111, 112) électromagnétique dans une direction prédéfinie et un récepteur pour la réception d'une réflexion du faisceau de balayage provenant de la direction prédéfinie, et l'unité d'évaluation (30) est réalisée pour déterminer à partir de la réception d'un signal émis par chaque unité de balayage et réfléchi et du temps de propagation de celui-ci un signal de temps de propagation (120a-d, 130a-d) et pour élaborer à partir des signaux de temps de propagation reçus pendant une période un profil de temps de propagation pour chacune des unités de balayage.

3. Dispositif de surveillance d'animaux selon la revendication 1,
**caractérisé en ce que** l'unité d'évaluation (30) comprend une mémoire de profil de temps de propagation électronique et est réalisée en outre pour
- mettre en mémoire dans la mémoire de profil de temps de propagation au moins un modèle de profil de temps de propagation prédéfini et une indication de lieu associée à ce modèle de profil de temps de propagation prédéfini,
- élaborer à partir d'une pluralité de signaux de temps de propagation déterminés pendant une période de balayage un profil de temps de propagation, lequel représente les signaux de temps de propagation (120a-d, 130a-d) pendant la période de balayage,
- comparer au moins une section du profil de temps de propagation au modèle de profil de temps de propagation prédéfini mis en mémoire dans la mémoire de profil de temps de propagation, et
- lorsqu'une concordance de la au moins une section du profil de temps de propagation avec le modèle de profil de temps de propagation est constatée, associer à la partie provenant du profil de temps de propagation l'indication de lieu mise en mémoire dans la mémoire de profil de temps de propagation par rapport au modèle de profil de temps de propagation prédéfini.

4. Dispositif de surveillance d'animaux selon la revendication 3,
**caractérisé en ce que** l'unité d'évaluation (30) est réalisée pour
- lors de la comparaison de la au moins une section du profil de temps de propagation au modèle de profil de temps de propagation prédéfini mis en mémoire dans la mémoire de profil de temps de propagation comparer une deuxième partie de profil de temps de propagation et un écart de lieu, qui définit une distance spatiale entre la première partie de profil de temps de propagation et la deuxième partie de profil de temps de propagation, à une première partie de modèle de profil de temps de propagation du modèle de profil de temps de propagation, à une deuxième partie de modèle de profil de temps de propagation du modèle de profil de temps de propagation et à un écart de lieu prédéfini, qui définit une distance entre la première partie de modèle de profil de temps de propagation et la deuxième partie de modèle de profil de temps de propagation, et
- lorsqu'une concordance de la première partie de profil de temps de propagation avec la première partie de modèle de profil de temps de propagation est constatée et qu'une concordance de la deuxième partie de profil de temps de propagation avec la deuxième partie de modèle de profil de temps de propagation est constatée et qu'une concordance de l'écart de lieu avec l'écart de lieu prédéfini est constatée, associer au profil de temps de propagation l'indication de lieu mise en mémoire dans la mémoire de profil de temps de propagation par rapport au modèle de profil de temps de propagation prédéfini.

5. Dispositif de surveillance d'animaux selon l'une quelconque des revendications précédentes,
**caractérisé en ce que** le rayonnement électromagnétique est un rayonnement laser.

6. Dispositif de surveillance d'animaux selon l'une quelconque des revendications précédentes,
**caractérisé par** un système de déplacement pour le déplacement du dispositif de balayage le long d'un système de retenue d'animaux dans une direction de déplacement (A), qui est orientée transversalement, en particulier perpendiculairement au dispositif de balayage.

7. Dispositif de surveillance d'animaux selon la revendication 6,
**caractérisé en ce que** l'unité d'évaluation (30) est réalisée pour détecter un profil de temps de propagation pendant un mouvement continu du dispositif de balayage le long du système de retenue d'animaux, dans lequel le système de retenue d'animaux présente de préférence une pluralité d'unités de retenue d'animaux séparées, détachées les unes des autres, et l'unité d'évaluation est réalisée pour détecter la pluralité des unités de retenue d'animaux séparées, détachées les unes des autres pendant le mouvement de déplacement dans un seul profil de temps de propagation et pour le comparer à un modèle de profil de temps de propagation mis en mémoire pour la pluralité des unités de retenue d'animaux séparées, détachées les unes des autres.

8. Dispositif de surveillance d'animaux selon l'une quelconque des revendications 6 ou 7,
**caractérisé en ce que** dans la direction de déplacement (A) une unité de balayage voisine (10a) est disposée de manière espacée d'un écart et par rapport à la direction de déplacement en alignement avec une unité de balayage, dans lequel
- l'unité d'évaluation (30) est réalisée pour déterminer à partir de la réception d'un signal émis par l'unité de balayage voisine (10a) et du temps de propagation de celui-ci un signal de temps de propagation voisin
- l'unité de balayage et l'unité de balayage voisine avec l'unité d'évaluation sont réalisées et reliées pour une détection simultanée d'un profil de temps de propagation à partir du signal de temps de propagation (120a-d, 130a-d) et d'un profil de temps de propagation voisin à partir du signal de temps de propagation voisin, et
- l'unité d'évaluation (30) est réalisée pour comparer le profil de temps de propagation et le profil de temps de propagation voisin l'un à l'autre avec prise en compte de l'écart et d'une vitesse de déplacement et pour évaluer des signaux de temps de propagation, qui ne se trouvent pas de manière concordante dans le profil de temps de propagation et le profil de temps de propagation voisin, comme des signaux de vie.

9. Dispositif de surveillance d'animaux selon la revendication 8,
**caractérisé en ce que** l'unité de balayage voisine (10a) envoie et reçoit dans un dispositif de balayage voisin, qui s'étend de manière non parallèle au dispositif de balayage de l'unité de balayage et qui se situe dans un plan plat avec le dispositif de balayage et la direction de déplacement (A),
dans lequel le dispositif de balayage voisin est orienté de préférence de telle sorte que dans un plan de référence, qui est orienté perpendiculairement à la direction de déplacement, il se tient à un angle par rapport à la verticale, qui concorde avec l'angle dans lequel le système de balayage se tient à la verticale dans ce plan de référence,
et que l'unité d'évaluation (30) est réalisée pour comparer sur la base du signal de temps de propagation, du signal de temps de propagation voisin, de l'écart entre l'unité de balayage et l'unité de balayage voisine et de l'angle entre le dispositif de balayage et le dispositif de balayage voisin le profil de temps de propagation et le profil de temps de propagation voisin l'un à l'autre avec prise en compte de l'écart et de la vitesse de déplacement et pour évaluer des signaux de temps de propagation, qui ne se trouvent pas de manière concordante dans le profil de temps de propagation et le profil de temps de propagation voisin, comme des signaux de vie.

10. Dispositif de surveillance d'animaux selon l'une quelconque des revendications précédentes,
**caractérisé en ce que** le rayonnement électromagnétique présente une longueur d'onde dans une plage de longueurs d'onde non visible pour une poule.

11. Dispositif de surveillance d'animaux selon l'une quelconque des revendications précédentes,
**caractérisé en ce que** chaque unité de balayage (10, 11, 12) envoie un faisceau électromagnétique unidimensionnel et constant dans son orientation angulaire.

12. Dispositif de surveillance d'animaux selon l'une quelconque des revendications précédentes,
**caractérisé en ce que** chaque unité de balayage (10, 11, 12) envoie un faisceau électromagnétique puisé.

13. Dispositif de surveillance d'animaux selon l'une quelconque des revendications précédentes,
**caractérisé par** un capteur de température à mesure sans contact, qui est disposé pour une mesure de température le long du dispositif de balayage et au voisinage ou de manière coaxiale par rapport au dispositif de balayage.

14. Dispositif de surveillance d'animaux selon la revendication 13,
**caractérisé en ce que** le capteur de température est relié par technique de signaux à l'unité d'évaluation (30) et l'unité d'évaluation est réalisée pour associer l'un à l'autre un signal détecté avec le dispositif de balayage et un signal détecté simultanément avec le capteur de température.

15. Dispositif de surveillance d'animaux selon la revendication 1,
**caractérisé en ce que** l'unité d'évaluation (30) est réalisée pour déterminer sur la base de l'écart régulier dans le temps déterminé par le système de comptage et/ou de l'espacement régulier dans l'espace déterminé par le système de comptage des structures détectées par le système de balayage une position du système de balayage le long d'un trajet de déplacement et pour associer cette position au profil de temps de propagation.

16. Dispositif de surveillance d'animaux selon l'une quelconque des revendications précédentes,
**caractérisé par** un capteur couplé à un élément de transmission entraîné par une unité d'entraînement (10, 11, 12) ou à un élément de transmission entraîné de manière passive, en particulier une roue d'entraînement ou une roue de friction pour la détection de la distance de trajet de l'élément de transmission, dans lequel l'unité d'évaluation est couplée au capteur par technique de signaux et réalisée pour déterminer à partir d'un signal de distance de trajet reçu par le capteur une position du système de balayage le long d'un trajet de déplacement et pour associer cette position au profil de temps de propagation.
